(19) Europäisches Patentamt
European Patent Office
Office européen des brevets

(11) Numéro de publication: **0 321 368**
**A1**

# (12) DEMANDE DE BREVET EUROPEEN

(21) Numéro de dépôt: **88420420.7**

(22) Date de dépôt: **15.12.88**

(51) Int. Cl.[4]: **C 07 K 5/06**
A 23 L 1/236

(30) Priorité: **18.12.87 FR 8718113**

(43) Date de publication de la demande:
**21.06.89 Bulletin 89/25**

(84) Etats contractants désignés:
**AT BE CH DE ES FR GB GR IT LI LU NL SE**

(71) Demandeur: **UNIVERSITE CLAUDE BERNARD - LYON 1**
**43, boulevard du 11 Novembre 1918**
**F-69100 Villeurbanne Cédex (FR)**

(72) Inventeur: **Nofre, Claude**
**119 cours Albert Thomas**
**F-69003 Lyon (FR)**

**Tinti, Jean-Marie**
**5 avenue de Grenoble**
**F-69330 Meyzieu (FR)**

(74) Mandataire: **Laurent, Michel et al**
**Cabinet LAURENT et GUERRE B.P. 32**
**F-69131 Ecully Cedex (FR)**

Revendications pour les Etats contractants suivants: ES + GR.

(54) **Edulcorants dérivés hétérocycliques de N-carbamoyl-, N-thiocarbamoyl- ou N-amidino-aminomalonyl ou aminosuccinyl amides.**

(57) Agents édulcorants de formule :

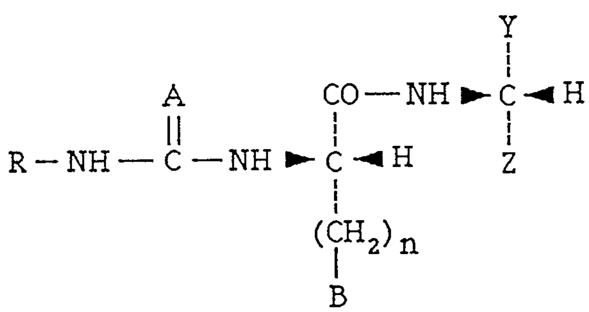

dans laquelle R est un groupe hétérocyclique qui, dans une forme de réalisation préférée, est un groupe 2-cyanopyrid-5-yle ou 2-cyanopyrimidin-5-yle, A est O, S ou NCN, n est 0 ou 1, B est COOH, et dans laquelle, dans une forme de réalisation préférée, Y est $COOCH_3$ et Z est $CH_2C_6H_5$.

EP 0 321 368 A1

**Description**

**EDULCORANTS DERIVES HETEROCYCLIQUES DE N-CARBAMOYL-, N-THIOCARBAMOYL- OU N-AMIDINO-AMINOMALONYL OU AMINOSUCCINYL AMIDES.**

La présente invention concerne de nouveaux agents édulcorants utiles, notamment, pour édulcorer les aliments, les boissons, les confiseries, les pâtisseries, le chewing gum, les produits d'hygiène, les cosmétiques, les articles de toilette, les produits pharmaceutiques et vétérinaires, et leurs équivalents. Elle concerne aussi des préparations et des compositions contenant de tels agents édulcorants.

Dans le brevet EP-B-0107597 on a décrit comme nouveaux agents édulcorants des composés de formule :

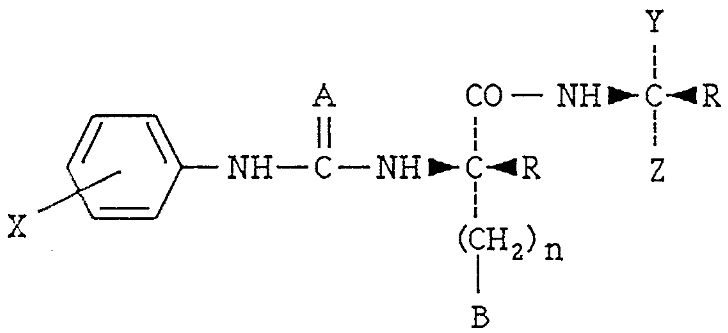

dans laquelle :
- X représente au moins un groupe donneur de doublets libres ;
- A représente un atome d'oxygène ou de soufre ou un groupe imino ou méthylène ;
- R représente un atome d'hydrogène ou un groupe alkyle de 1 à 4 atomes de carbone ;
- n représente un nombre égal à 0 ou 1 ;
- B représente un groupe COOH ou COOM (M désignant un cation) ;
- Y représente soit un groupe donneur de doublets libres, soit un groupe alkyle R′, R′ désignant un groupe alkyle de I à 4 atomes de carbone ;
- Z représente :

. soit un groupe hydrophobe R″, R″ désignant un groupe alkyle, cycloalkyle, benzyle ou aryle ayant jusqu'à 12 atomes de carbone,

. soit un groupe hydrophobe R″ dans lequel 1 ou 2 atomes de carbone sont substitués par des atomes d'oxygène ou de soufre,

. soit un groupe hydrophobe R″ fixé par un groupe amidique ou ester, ou par un carbone halogéné ou alcoolique.

Dans le brevet EP-A-0195731 on a proposé comme nouveaux agents édulcorants des composés de formule :

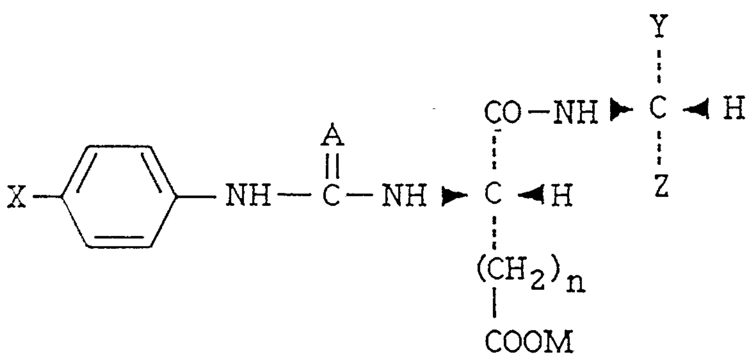

dans laquelle :
- X est un substituant CN, COOC$_1$-C$_3$ alkyle, COC$_1$-C$_3$ alkyle, CONHC$_1$-C$_3$ alkyle, SO$_2$C$_1$-C$_3$ alkyle, SOC$_1$-C$_3$ alkyle, SO$_2$NHC$_1$-C$_3$ alkyle, NO$_2$, F, Cl ;
- A est un groupe N-CN ;

- n est un nombre égal à 0 ou à 1 ;
- M est un atome d'hydrogène, ou un cation organique ou inorganique physiologiquement acceptable ;
- Y est un substituant $COOC_1$-C3 alkyle, $C_1$-$C_3$ alkyle, $CH_2OH$, $CHOHCH_3$, $CF_3$ ;
- Z est un groupe $C_1$-$C_5$ *n*-alkyle, isobutyle, isopentyle, phényle, cyclohexyle, benzyle, cyclohexyl méthyle, $CH_2C_6H_4OH$(*p*), $CH_2OC_1$-$C_4$ alkyle, $CH_2COOC_1$-$C_4$ alkyle, $CH_2SC_1$-$C_4$ alkyle, $CH_2CH_2SCH_3$, $CH_2CH_2SO_2CH_3$, $COOC_1$-$C_4$ alkyle, $COOC_3$-$C_7$ cycloalkyle, $CONHC_2$-$C_4$ alkyle, $CONHC_3$-$C_7$ cycloalkyle, $CONHC_3$-$C_7$ thiacycloalkyle, CONH-$CH_2COOCH_3$.

Or, on a observé que les composés édulcorants décrits dans les documents précédemment cités sont insuffisamment solubles dans l'eau, notamment à pH acide, pour que l'on puisse envisager leur utilisation dans les boissons gazeuses ("soft drinks") qui constituent le principal débouché des édulcorants de synthèse.

Le perfectionnement qu'apporte la présente invention remédie à cet inconvénient.

La présente invention concerne des agents édulcorants de formule :

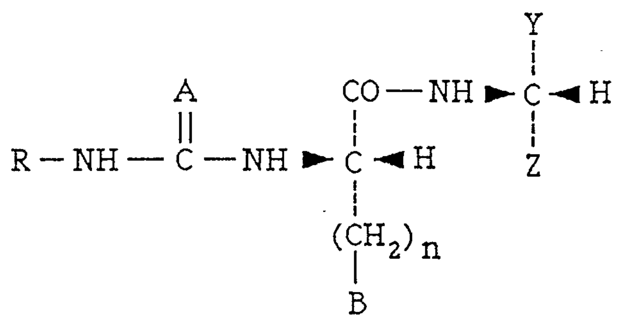

dans laquelle :
- A est O, S ou N-CN ;
- n est 0 ou 1 ;
- B est un groupe COOH ou un sel de sodium, potassium, ammonium, calcium ou magnésium ;
- Y est choisi dans le groupe constitué par :

$C_1$-$C_3$ alkyle,
$CH_2OH$,
$CHOHCH_3$,
$COOC_1$-$C_3$ alkyle,
2-furyle ou $CH_2OCH_3$ ;
- Z est :

. un groupe hydrocarboné $C_2$-$C_{13}$, saturé ou insaturé, acyclique, cyclique ou mixte, ou

. un groupe hydrocarboné $C_2$-$C_{13}$ modifié, saturé ou insaturé, acyclique, cyclique ou mixte, dans lequel jusqu'à quatre C, CH ou $CH_2$ peuvent être remplacés par un à quatre hétéroatomes ou radicaux hétéroatomiques, identiques ou différents, choisis dans le groupe comprenant N, O, S ou NH, C étant remplacé par S, CH étant remplacé par N, et $CH_2$ étant remplacé par O, S ou NH ;

caractérisés en ce que R est un groupe hetérocyclique monocyclique ou bicyclique,

. le groupe monocyclique étant choisi dans le groupe constitué par les radicaux :

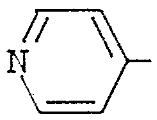

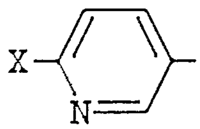

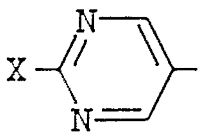

dans lesquels X est :
H,
Cl,
CN,
$COC_1$-$C_3$ alkyle,
$CONHC_1$-$C_3$ alkyle,
$COOC_1$-$C_3$ alkyle,
F,
$NO_2$,
$OCH_3$,
$SOC_1$-$C_3$ alkyle,
$SO_2C_1$-$C_3$ alkyle ou
$SO_2NHC_1$-$C_3$ alkyle,
. le groupe bicyclique étant choisi dans le groupe constitué par les radicaux :

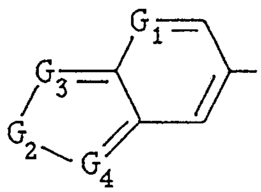

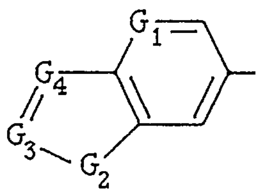

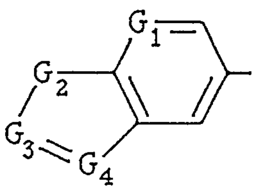

:

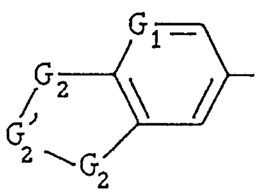

dans lesquels :
$G_1$ est CH ou N,
$G_2$ et $G_2'$ sont $CH_2$, CO, NH, $NCH_3$, O, S ou $SO_2$,
$G_3$ et $G_4$ sont CH, $CCH_3$, N ou NO.

En d'autres termes, les composés de la présente invention se distinguent des composés décrits dans les brevets cités dans le préambule par le remplacement du groupe carbocyclique ($X\text{-}C_6H_4$) par un groupe hétérocyclique (R), ce qui a pour effet d'accroître leur solubilité dans l'eau, surtout en milieu acide, aux pH d'utilisation des boissons gazeuses (pH 2,5 - 3). De plus, le remplacement d'un groupe carbocyclique $X\text{-}C_6H_4$ par un groupe hétérocyclique R ne modifie que peu le pouvoir édulcorant des composés, ce qui est totalement inattendu quand on sait que toute modification, même légère, de la structure moléculaire d'un agent édulcorant entraîne souvent une suppression du caractère édulcorant, les relations entre la structure et l'activité édulcorante étant en effet imprévisibles (M.G.J. BEETS, Structure-Activity Relationships in Human Chemoreception, Applied Science Publ., London, 1978, p. 259-362 ; H. VAN DER WEL, A. VAN DER HEIJDEN, H. G. PEER, Food Reviews International, 1987, 3, 193-268).

Avantageusement, dans les agents édulcorants selon l'invention :
- R est un groupe 2-cyanopyrid-5-yle ou 2-cyanopyrimidin-5-yle ;
- A est O ;
- n est 1 ;
- Y est $COOCH_3$ ou $CH_3$ ;
- Z est un groupe butyle, pentyle, isobutyle, isopentyle, phényle, cyclohexyle, benzyle, cyclohexylméthyle, propylamide, 2-butylamide, dicyclopropylcarbinylamide ou 2,2,4,4-tétraméthylthiéthan-3-ylamide.

L'agent édulcorant préféré de l'invention est :

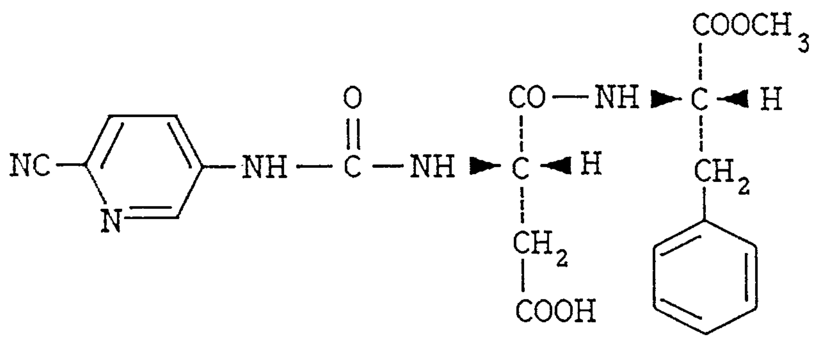

L'invention concerne également le procédé qui consiste à édulcorer les aliments, boissons, confiseries, pâtisseries, chewing gum, produits d'hygiène, cosmétiques, articles de toilette, produits pharmaceutiques et vétérinaires, et leurs équivalents, en leur ajoutant une quantité adéquate d'un ou plusieurs agents édulcorants selon la présente invention. Par "quantité adéquate", on désigne une quantité d'agent édulcorant suffisante pour produire la perception d'une saveur sucrée.

L'invention concerne aussi les préparations édulcorées suivant le procédé de la présente invention.

L'invention concerne également les compositions édulcorantes caractérisées en ce qu'elles comprennent une quantité adéquate d'au moins un agent édulcorant selon la présente invention, et un support ou agent de charge approprié.

L'invention concerne aussi les compositions édulcorantes caractérisées en ce qu'elles comprennent une quantité adéquate d'un ou plusieurs agents édulcorants selon la présente invention, et un ou plusieurs autres agents édulcorants.

Les produits susceptibles d'être édulcorés par les agents édulcorants de la présente invention comprennent tous les produits pour lesquels on désire un composant de gout sucré, notamment, et ce de manière non limitative, les produits alimentaires (pour la consommation humaine ou animale), les boissons (boissons alcooliques, boissons non alcooliques, jus, boissons gazeuses), les confiseries, les pâtisseries, le chewing gum, les produits d'hygiène, les cosmétiques, les produits pharmaceutiques et vétérinaires, et leurs équivalents.

Les agents édulcorants de la présente invention peuvent être ajoutés sous forme pure aux produits comestibles pour leur communiquer un goût sucré. Toutefois, par suite du pouvoir sucrant élevé des présents agents édulcorants, ils sont généralement mélangés à un support ("carrier") ou à un agent de charge approprié ("bulking agent"). Avantageusement, les supports ou agents de charge appropriés sont choisis dans le groupe constitué par le polydextrose, l'amidon, les maltodextrines, la cellulose, la méthylcellulose, la carboxyméthylcellulose, l'hydroxyméthylcellulose, la cellulose microcristalline, l'alginate de sodium, les pectines, les gommes, le lactose, le maltose, le glucose, la leucine, le glycérol, le mannitol, le sorbitol, le bicarbonate de sodium, les acides phosphorique, citrique, tartrique, fumarique, benzoïque, sorbique, propionique, et leurs sels de sodium, potassium et calcium, et leurs équivalents.

Les présents agents édulcorants peuvent, dans un produit comestible, être employés seuls, comme unique agent édulcorant, ou sous forme de mélanges de deux ou plusieurs agents édulcorants de la présente invention. Les présents agents édulcorants peuvent, en outre, être utilisés en combinaison avec d'autres agents édulcorants tels que les sucres (saccharose), le sirop de maïs, le fructose, les dérivés dipeptidiques sucrés (aspartame, alitame), la néhohespéridine dihydrochalcone, l'isomaltulose hydrogéné, le stévioside, les sucres L, la glycyrrhizine, le xylitol, le sorbitol, le mannitol, l'acésulfame-K, la saccharine et ses sels de sodium, potassium, ammonium et calcium, l'acide cyclamique et ses sels de sodium, potassium, ammonium et calcium, le trichlorogalactosucrose, la monelline, la thaumatine, et leurs équivalents.

Les composés de l'invention peuvent être préparés par condensation entre un composé de formule :

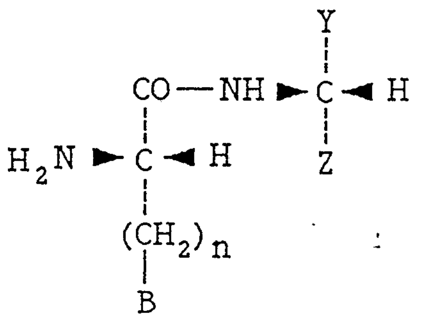

:

et un composé de formule :

$R - N=C=A$

dans laquelle A est O ou S,

ou un composé de formule :

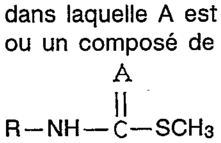

dans laquelle A est NCN.

Lorsque A est O ou S, la réaction peut être commodément réalisée dans l'eau à température ambiante. Le dérivé $R\text{-}N=C=A$ peut être préalablement dissous dans un solvant comme le benzène ou le chlorobenzène, ce qui améliore notablement le rendement de la réaction. Lorsque A est NCN, la réaction est de préférence effectuée à ébullition dans un mélange éthanol-eau. Dans les deux cas la condensation est réalisée en milieu basique, en présence de NaOH, KOH, $Na_2CO_3$, $NaHCO_3$ ou $N(C_2H_5)_3$.

Les composés de l'invention peuvent exister, sous forme acide ou sous forme de sel. Ils peuvent donc etre salifiés par des bases inorganiques ou organiques physiologiquement acceptables. Les sels préférés de l'invention sont les sels de sodium, potassium, ammonium calcium et magnésium. L'une des méthodes de choix pour préparer ces sels consiste à concentrer à sec, sous vide, un mélange, en solution aqueuse, d'un composé de l'invention et d'un équivalent d'un composé basique approprié.

La purification des composés de l'invention a été réalisée selon les techniques standards (recristallisation, chromatographie). Leur pureté et leur structure ont été vérifiées par les techniques classiques (chromatographie sur couche mince, chromatographie liquide haute performance, spectrométrie infra-rouge, résonance magnétique nucléaire, analyse élémentaire).

Le pouvoir édulcorant des composés ainsi préparés a été évalué par un groupe de huit goûteurs expérimentés. Pour cela, les composés, en solution aqueuse à des concentrations variables, sont comparés, sur le plan gustatif, à une solution témoin de saccharose à 2 % et dans certains cas à 5 % et 10 %, c'est-à-dire à des concentrations correspondant à celles utilisées lors d'un usage courant. Le pouvoir sucrant des édulcorants de synthèse varie en effet suivant la concentration de la solution de saccharose utilisée comme référence. Le pouvoir édulcorant du composé testé par rapport au saccharose correspond alors au rapport pondéral qui existe entre le composé et le saccharose à égale intensité édulcorante, c'est-à-dire quand les saveurs sucrées de la solution du composé testé et de la solution témoin de saccharose sont considérées, par une majorité de goûteurs, avoir la meme intensité édulcorante.

Les agents édulcorants de la présente invention ont pour utilité de pouvoir être ajoutés à tout produit comestible dans lequel on désire apporter un goût sucré, à condition qu'on les ajoute en proportions suffisantes pour atteindre le niveau d'édulcoration désiré. La concentration optimale d'utilisation de l'agent édulcorant dépendra de facteurs divers tels que, par exemple, le pouvoir sucrant de l'agent édulcorant, les conditions de stockage et d'utilisation des produits, les constituants particuliers des produits, le profil gustatif des produits comestibles et le niveau d'édulcoration désiré. Toute personne du métier peut facilement déterminer la proportion optimale d'agent édulcorant qui doit être employée pour l'obtention d'un produit comestible en réalisant des analyses sensorielles de routine. Les agents édulcorants préférés de la présente invention sont, en général, ajoutés aux produits comestibles dans des proportions d'environ 0,0001 à environ 0,002 pour cent en poids du produit comestible. Les produits concentrés contiendront évidemment des pourcentages plus élevés d'agent(s) édulcorant(s), et seront ensuite dilués suivant les intentions finales d'utilisation.

La présente invention permet donc d'accéder à des agents édulcorants qui sont parmi les plus puissants obtenus jusqu'ici, puisque, par exemple, le composé préféré de l'invention est 8 000 fois plus sucré que le saccharose, sans commune mesure donc avec le pouvoir sucrant des principaux agents édulcorants actuellement utilisés, proposés, développés ou en cours d'expérimentation, et qui sont rappelés dans le tableau I ci-après.

TABLEAU I

| Agents édulcorants | Pouvoir sucrant (saccharose = 1) |
|---|---|
| Xylitol | 1 |
| Cyclamate de sodium | 30 |
| Glycyrrhizine | 50 |
| Acésulfame-K | 200 |
| Aspartame | 200 |
| Stévioside | 300 |
| Saccharine | 400 |
| Néohespéridine dihydrochalcone | 1000 |
| Trichlorogalactosu-crose | 2000 |
| Alitame | 2000 |
| Thaumatine | 2000 |
| Monelline | 2000 |

Un autre avantage très important des agents édulcorants de la présente invention est de donner souvent une saveur sucrée très proche de celle du saccharose, notamment sans arrière-goût métallique ou amer (comme c'est le cas par exemple pour la saccharine ou l'acésulfame-K).

Les dérivés hétérocycliques décrits dans la présente invention, tout en ayant une stabilité en milieu acide comparable à celle de leurs analogues carbocycliques décrits dans l'art antérieur (brevets EP-B-0107597 et EP-A-0195731) ont l'avantage, comme déjà dit, d'être beaucoup plus solubles dans l'eau, comme l'étude comparée de leur solubilité a pu le montrer.

La solubilité a été mesurée de manière suivante. On met en suspension dans l'eau à pH 3 (tampon phosphate) et à température voisine de 0° C (ce qui correspond aux conditions de préparation et de stockage des boissons gazeuses) un excès du composé dont veut déterminer la solubilité. On soumet alors la suspension, toujours dans un bain de glace, à l'action des ultrasons pendant 60 min, puis on filtre (sur filtre Millipore Millex-HV 0,45 micromètre). Le composé, qui se trouve ainsi dissous à saturation dans le filtrat, est dosé par chromatographie liquide haute performance (HPLC) par rapport à une solution de référence de concentration connue.

C'est ainsi qu'on a, par exemple, observé que le composé décrit dans l'exemple 4 de la présente invention (composé dans lequel R est un groupe 2-cyanopyrid-5-yle) a une solubilité à 0° C, d'environ 70 mg/L, pendant que, dans les mêmes conditions, son analogue carbocyclique 4 cyanophényle (exemple 4 du brevet EP-B-0107597) n'a qu'une solubilité d'environ 20 mg/L, la solubilité du dérivé hétérocyclique étant donc environ 3,5 fois plus élevée que celle de son analogue carbocylique. De même, on a observé par exemple que le composé décrit dans l'exemple 8 de la présente invention (composé dans lequel R est un groupe 5-benzofurazanyle) a une solubilité, à 0° C, d'environ 180 mg/L, soit environ 9 fois plus que celle du composé carbocyclique 4-cyanophényle (exemple 4 du brevet EP-B-0107597).

La manière dont l'invention peut être réalisée et les avantages qui en découlent ressortiront mieux des exemples de réalisation qui suivent.

<u>EXEMPLE 1 :</u>

Synthèse du N-(3-pyridylcarbamoyl)-L-aspartyl-L-phénylalanine méthyl ester :

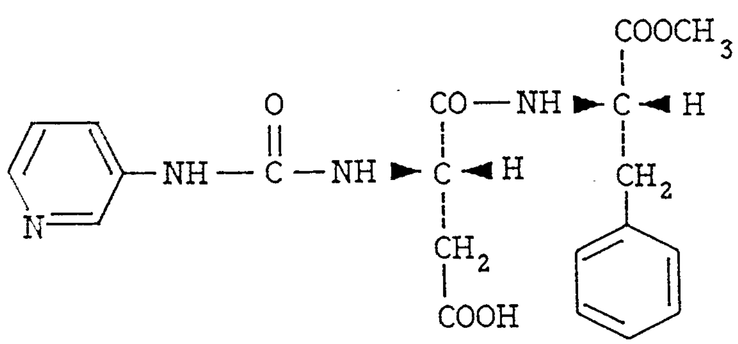

A une solution de 25 $cm^3$ d'eau contenant 7,6 g (25,8 mmol) de L-aspartyl-L-phénylalanine méthyl ester et 1,64 g (15,4 mmol) de carbonate de sodium anhydre, est ajoutée une solution de 3,1 g (25,8 mmol) de 3-pyridyl isocyanate dissous dans 10 $cm^3$ de benzène. Une agitation vigoureuse est maintenue durant 15 minutes à température ambiante avant de procéder à l'extraction du mélange par 3 x 50 $cm^3$ d'éther éthylique. La phase aqueuse est refroidie puis acidifiée par une solution d'acide chlorhydrique 3 N jusqu'à l'obtention d'un pH d'environ 4,5. Le précipité formé est filtré, lavé par 10 $cm^3$ d'eau froide puis séché. On obtient 3,4 g (rendement 32 %) d'un solide qui se présente sous la forme d'une poudre amorphe dont le point de fusion est de 186 °C.

Le pouvoir édulcorant de ce composé correspond approximativement, sur une base pondérale, à 100 (cent) fois celui du saccharose (par comparaison avec une solution de saccharose à 2 %).

EXEMPLE 2 :

Synthèse du N-(3-pyridylthiocarbamoyl)-L-aspartyl-L-phénylalanine méthyl ester :

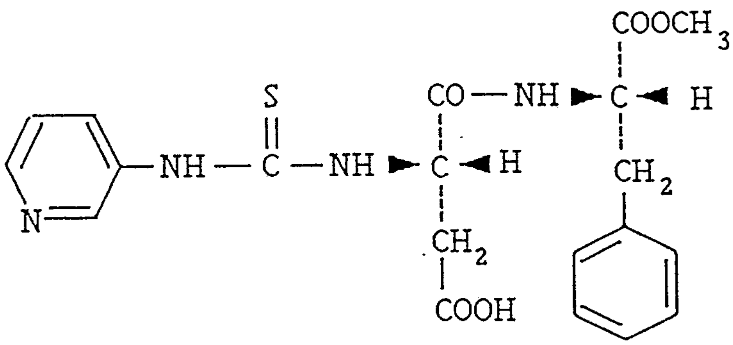

Ce composé est obtenu à partir du L-aspartyl-L-phénylalanine méthyl ester et du 3-pyridyl isothiocyanate, en suivant le protocole expérimental décrit dans l'exemple 1 (rendement 50 % ; point de fusion 145 °C).

Le pouvoir édulcorant de ce composé correspond approximativement, sur une base pondérale, à 1 000 (mille) fois celui du saccharose (par comparaison avec une solution de saccharose à 2 %).

EXEMPLE 3 :

Synthèse du N-(2-chloro-5-pyridylcarbamoyl)-L-aspartyl-L-phénylalanine méthyl ester :

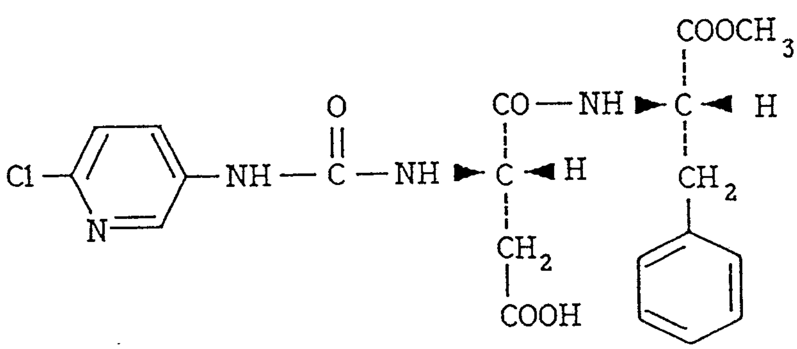

Ce composé est obtenu à partir du L-aspartyl-L-phénylalanine méthyl ester et du 2-chloro-5-pyridyl isocyanate, en suivant le protocole expérimental décrit dans l'exemple 1 (rendement 17 % ; point de fusion 184 °C).

Le pouvoir édulcorant de ce composé correspond approximativement, sur une base pondérale, à 500 (cinq cents) fois celui du saccharose (par comparaison avec une solution de saccharose à 2 %).

EXEMPLE 4 :

Synthèse du N-(2-cyano-5-pyridylcarbamoyl)-L-aspartyl-L-phénylalanine méthyl ester :

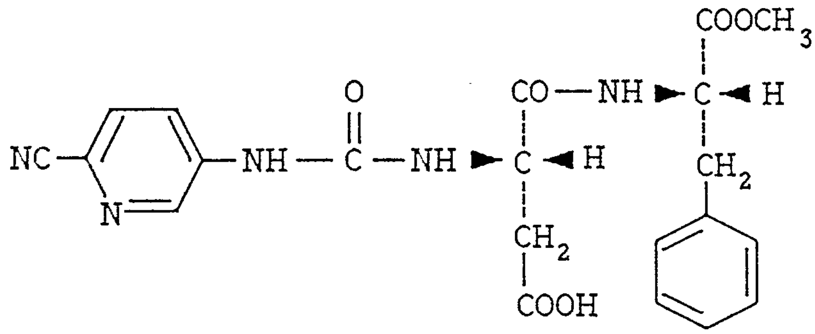

Ce composé est obtenu à partir du L-aspartyl-L-phénylalanine méthyl ester et du 2-cyano-5-pyridyl isocyanate, en suivant le protocole expérimental décrit dans l'exemple 1 (rendement 53 % ; point de fusion 203°C).

Le pouvoir édulcorant de ce composé correspond approximativement, sur une base pondérale, à 8 000 (huit mille) fois celui du saccharose à 2%, à 6 000 (six mille) fois par comparaison à une solution de saccharose à 5%, à 4 000 (quatre mille) fois par comparaison à une solution de saccharose à 10%.

EXEMPLE 5

Synthèse de l'acide N-(2-cyano-5-pyridylcarbamoyl)-L-aspartique N-[(RS)-alpha-(2-furyl)benzyl] alpha-monoamide :

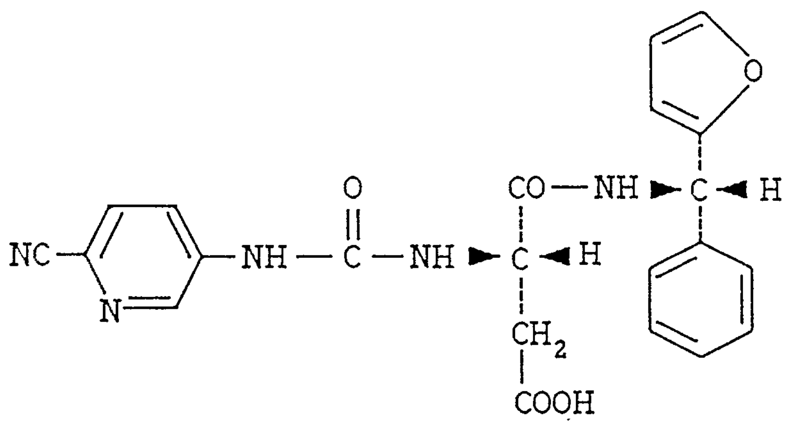

Ce composé est obtenu à partir de l'acide L-aspartique N-[DL-alpha-(2-furyl)benzyl)] alpha-monoamide et du 2-cyano-5-pyridyl isocyanate, en suivant le protocole expérimental décrit dans l'exemple 1 (rendement 60 % ; point de fusion 176°C).

Le pouvoir édulcorant de ce composé correspond approximativement, sur une base pondérale, à 700 (sept cents) fois celui du saccharose (par comparaison avec une solution de saccharose à 2 %).

EXEMPLE 6 :

Synthèse du N-(2-méthoxy-5-pyridylthiocarbamoyl)-L-aspartyl-L-phénylalanine méthyl ester :

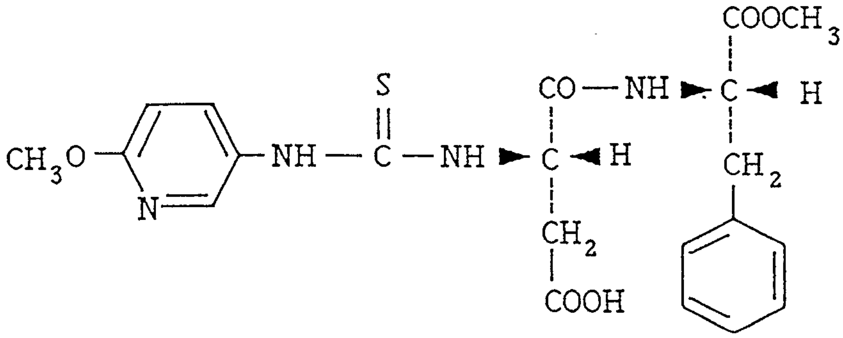

Ce composé est obtenu à partir du L-aspartyl-L-phénylalanine méthyl ester et du 2-méthoxy-5-pyridyl isothiocyanate, en suivant le protocole expérimental décrit dans l'exemple 1 (rendement 38 % ; point de fusion 161 °C).

Le pouvoir édulcorant de ce composé correspond approximativement, sur une base pondérale, à 1 400 (mille quatre cents) fois celui du saccharose (par comparaison avec une solution de saccharose à 2 %).

EXEMPLE 7 :

Synthèse du N-(2-cyano-5-pyrimidinylthiocarbamoyl)-L-aspartyl-L-phénylalanine méthyl ester :

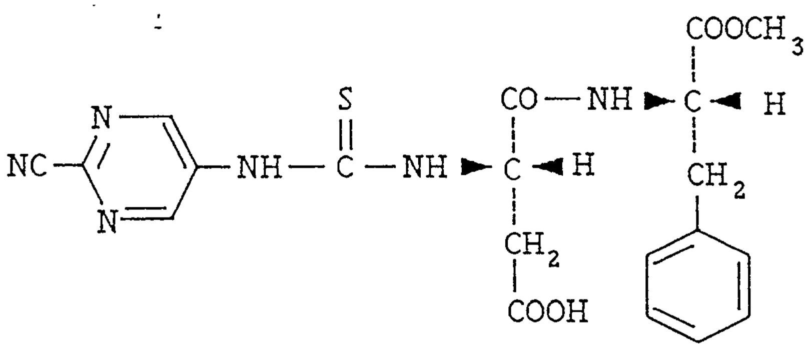

Ce composé est obtenu à partir du L-aspartyl-L-phénylalanine méthyl ester et du 2-cyano-5-pyrimidinyl isothiocyanate, en suivant le protocole expérimental décrit dans l'exemple 1 (rendement 8 % ; point de fusion 214 °C).

Le pouvoir édulcorant de ce composé correspond approximativement, sur une base pondérale, à 50 000 (cinquante mille) fois celui du saccharose (par comparaison avec une solution de saccharose à 2 %).

EXEMPLE 8 :

Synthèse du N-(5-benzofurazanylcarbamoyl)-L-aspartyl-L-phénylalanine méthyl ester :

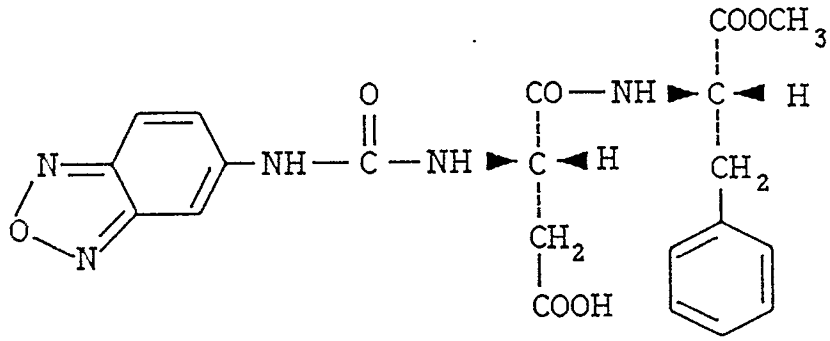

Ce composé est obtenu à partir du L-aspartyl-L-phénylalanine méthyl ester et du 5-benzofurazanyl isocyanate, en suivant le protocole expérimental décrit dans l'exemple 1 (rendement 25 % ; point de fusion 184 °C).

Le pouvoir édulcorant de ce composé correspond approximativement, sur une base pondérale, à 5 000 (cinq mille) fois celui du saccharose (par comparaison avec une solution de saccharose à 2 %).

EXEMPLE 9 :

Synthèse du N-(1-oxyde-5-benzofurazanylcarbamoyl)-L-aspartyl-L-phénylalanine méthyl ester :

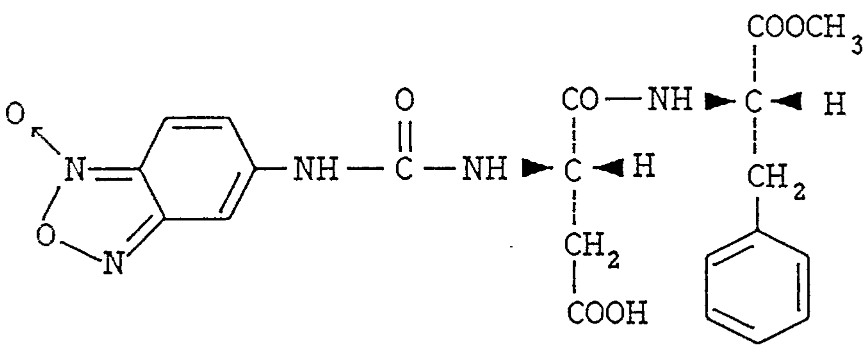

Ce composé est obtenu à partir du L-aspartyl-L-phénylalanine méthyl ester et du 1-oxyde-5-benzofurazanyl isocyanate, en suivant le protocole expérimental décrit dans l'exemple 1 (rendement 63 % ; point de fusion : 155° C, décomposition).

Le pouvoir édulcorant de ce composé correspond approximativement, sur une base pondérale, à 2 000 (deux mille) fois celui du saccharose (par comparaison avec une solution de saccharose à 2 %).

EXEMPLE 10 :

Synthèse du N-(5-benzothiofurazanylcarbamoyl)-L-aspartyl-L-phénylalanine méthyl ester :

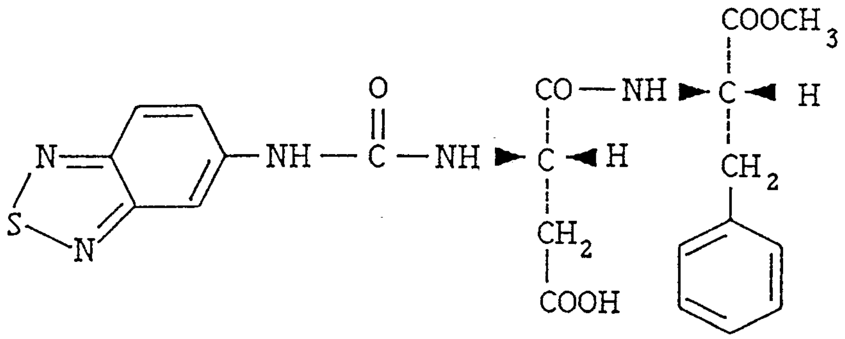

Ce composé est obtenu à partir du L-aspartyl-L-phénylalanine méthyl ester et du 5-benzothiofurazanyl isocyanate, en suivant le protocole expérimental décrit dans l'exemple 1 (rendement 25 % ; point de fusion 177° C).

Le pouvoir édulcorant de ce composé correspond approximativement, sur une base pondérale, à 500 (cinq cents) fois celui du saccharose (par comparaison avec une solution de saccharose à 2 %).

EXEMPLE 11 :

Synthèse du N-(3,4-méthylènedioxyphénylcarbamoyl)-L-aspartyl-L-phénylalanine méthyl ester :

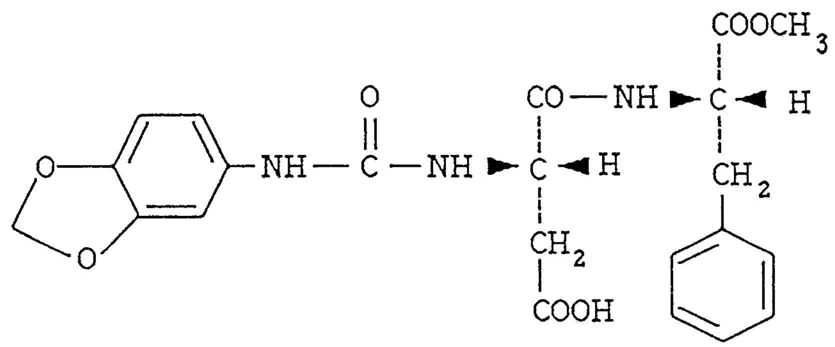

Ce composé est obtenu à partir du L-aspartyl-L-phénylalanine méthyl ester et du 3,4-méthylènedioxyphényl isocyanate, en suivant le protocole expérimental décrit dans l'exemple 1 (rendement 55 % ; point de fusion 189 °C).

Le pouvoir édulcorant de ce composé correspond approximativement, sur une base pondérale, à 50 (cinquante) fois celui du saccharose (par comparaison avec une solution de saccharose à 2 %).

Les pouvoirs édulcorants obtenus avec les différents composés cités dans les Exemples 1 à 11 sont récapitulés dans le Tableau II ci-après ; les pouvoirs édulcorants ainsi donnés ont été évalués, sur une base pondérale, par rapport à une solution de saccharose à 2 %.

## TABLEAU II

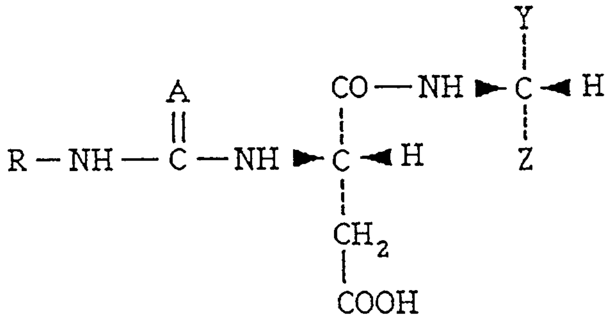

| Composé | R | A | Y | Z | Pouvoir édulcorant |
|---|---|---|---|---|---|
| 1 | 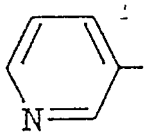 | O | $COOCH_3$ | $CH_2C_6H_5$ | 100 |
| 2 | 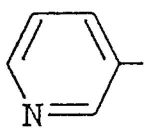 | S | $COOCH_3$ | $CH_2C_6H_5$ | 1 000 |
| 3 | 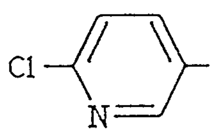 | O | $COOCH_3$ | $CH_2C_6H_5$ | 500 |
| 4 | 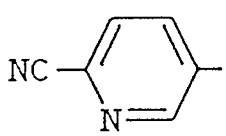 | O | $COOCH_3$ | $CH_2C_6H_5$ | 8 000 |
| 5 | 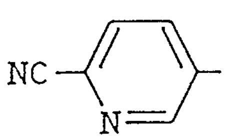 | O | 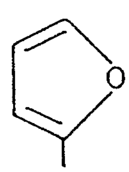 | $C_6H_5$ | 700 |

| Composé | R | A | Y | Z | Pouvoir édulcorant |
|---|---|---|---|---|---|
| 6 | 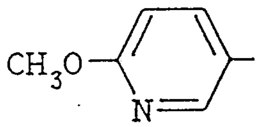 | S | $COOCH_3$ | $CH_2C_6H_5$ | 1 400 |
| 7 | 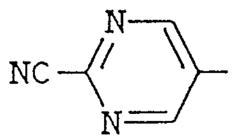 | S | $COOCH_3$ | $CH_2C_6H_5$ | 50 000 |
| 8 | 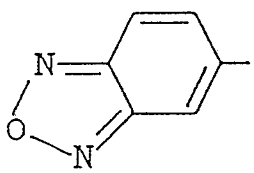 | 0 | $COOCH_3$ | $CH_2C_6H_5$ | 5 000 |
| 9 | 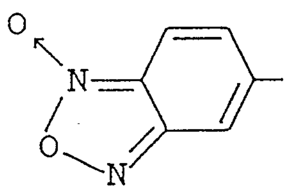 | 0 | $COOCH_3$ | $CH_2C_6H_5$ | 2 000 |
| 10 | 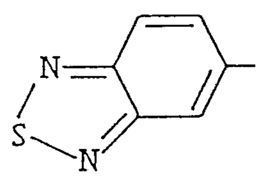 | 0 | $COOCH_3$ | $CH_2C_6H_5$ | 500 |
| 11 | 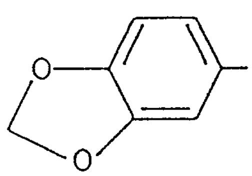 | 0 | $COOCH_3$ | $CH_2C_6H_5$ | 50 |

**Revendications**

1. Agents édulcorants de formule :

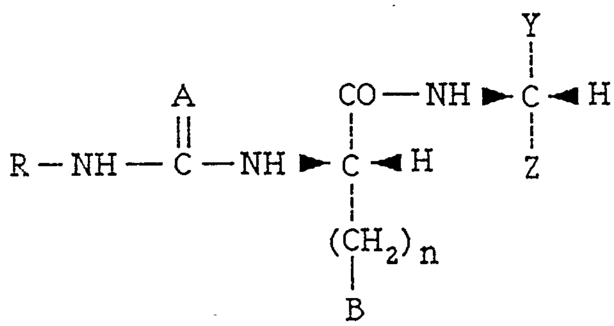

dans laquelle :
- A est O, S ou N-CN ;
- n est 0 ou 1 ;
- B est un groupe COOH ou un sel de sodium, potassium, ammonium, calcium ou magnésium ;
- Y est choisi dans le groupe constitué par :
$C_1$-$C_3$ alkyle,
$CH_2OH$,
$CHOHCH_3$,
$COOC_1$-$C_3$ alkyle,
2-furyle ou $CH_2OCH_3$ ;
- Z est :
. un groupe hydrocarboné $C_2$-$C_{13}$, saturé ou insaturé, acyclique, cyclique ou mixte, ou
. un groupe hydrocarboné $C_2$-$C_{13}$ modifié, saturé ou insaturé, acyclique, cyclique ou mixte, dans lequel jusqu'à quatre C, CH ou $CH_2$ peuvent être remplacés par un à quatre hétéroatomes ou radicaux hétéroatomiques, identiques ou différents, choisis dans le groupe comprenant N, O, S ou NH, C étant remplacé par S, CH étant remplacé par N, et $CH_2$ étant remplacé par O, S ou NH ;
caractérisés en ce que R est un groupe hétérocyclique monocyclique ou bicyclique,
. le groupe monocyclique étant choisi dans le groupe constitué par les radicaux :

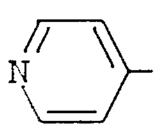

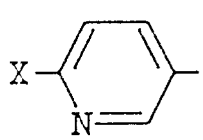

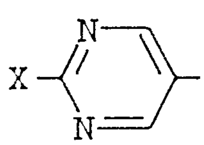

dans lesquels X est :
H,
Cl,

CN,
$COC_1$-$C_3$ alkyle,
$CONHC_1$-$C_3$ alkyle,
$COOC_1$-$C_3$ alkyle,
F,
$NO_2$,
$OCH_3$,
$SOC_1$-$C_3$ alkyle,
$SO_2C_1$-$C_3$ alkyle ou
$SO_2NHC_1$-$C_3$ alkyle,
. le groupe bicyclique étant choisi dans le groupe constitué par les radicaux :

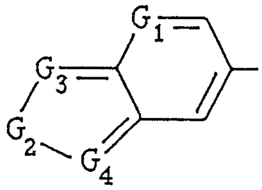

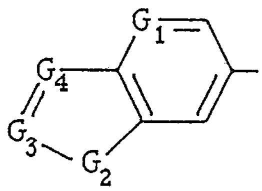

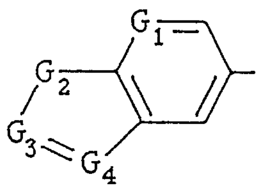

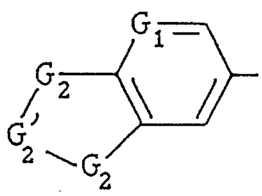

dans lesquels :
$G_1$ est CH ou N,
$G_2$ et $G_2'$ sont $CH_2$, CO, NH, $NCH_3$, O, S ou $SO_2$,
$G_3$ et $G_4$ sont CH, $CCH_3$, N ou NO.

2. Agents édulcorants selon la revendication 1 dans lesquels :
- R est un groupe 2-cyanopyrid-5-yle ou 2-cyanopyrimidin-5-yle ;
- A est O ;
- n est 1 ;
- Y est $COOCH_3$ ou $CH_3$ ;
- Z est un groupe butyle, pentyle, isobutyle, isopentyle, phényle, cyclohexyle, benzyle, cyclohexylméthyle, propylamide, 2-butylamide, dicyclopropylcarbinylamide ou 2,2,4,4-tétraméthylthiéthan-3-ylamide.

3. Agent édulcorant selon la revendication 1 caractérisé en ce qu'il consiste en un composé de formule :

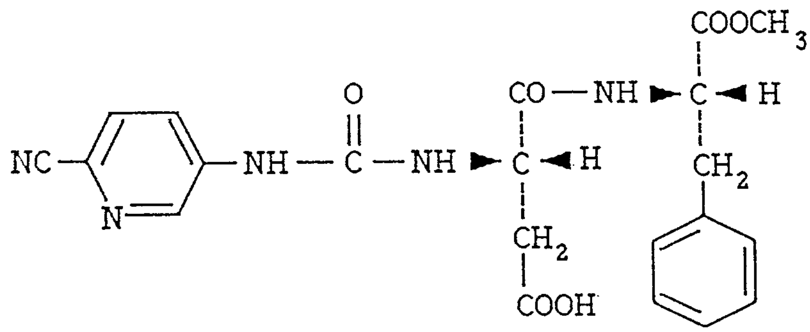

4. Procédé pour édulcorer les aliments, boissons, confiseries, patisseries, chewing gum, produits d'hygiène, cosmétiques, articles de toilette, produits pharmaceutiques et vétérinaires, caractérisé en ce qu'il consiste à ajouter à ces produits une quantité adéquate d'au moins un agent édulcorant selon la revendication 1.

5. Préparations édulcorées suivant le procédé de la revendication 4.

6. Compositions édulcorantes caractérisées en ce qu'elles comprennent une quantité adéquate d'un ou plusieurs agents édulcorants selon la revendication 1 et un support ou agent de charge choisi dans le groupe comprenant le polydextrose, l'amidon, les maltodextrines, la cellulose, la méthylcellulose, la carboxyméthylcellulose, l'hydroxyméthylcellulose, la cellulose microcristalline, l'alginate de sodium, les pectines, les gommes, le lactose, le maltose, le glucose, la leucine, le glycérol, le mannitol, le sorbitol, le bicarbonate de sodium et les acides phosphorique, citrique, tartrique, fumarique, benzoïque, sorbique et propionique, et leurs sels de sodium, potassium et calcium, et leurs équivalents.

7. Compositions édulcorantes caractérisées en ce qu'elles comprennent un agent édulcorant selon la revendication 1 et au moins un autre agent édulcorant, l'autre agent édulcorant étant choisi dans le groupe comprenant le saccharose, le sirop de maïs, le fructose, l'aspartame, l'alitame, la néohespéridine dihydrochalcone, l'isomaltulose hydrogéné, le stévioside, les sucres L, la glycyrrhizine, le xylitol, le sorbitol, le mannitol, l'acésulfame-K, la saccharine et ses sels de sodium, potassium, ammonium ou calcium, l'acide cyclamique et ses sels de sodium, potassium, ammonium ou calcium, le trichlorogalactosucrose, la monelline, la thaumatine et leurs équivalents.

**Revendications pour les Etats contractants suivants :**

1. Procédé pour la préparation d'agents édulcorants de formule :

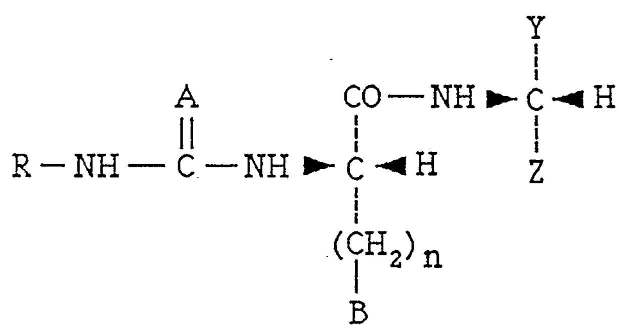

dans laquelle :
- A est O, S ou N-CN ;
- n est 0 ou l ;
- B est un groupe COOH ou un sel de sodium, potassium, ammonium, calcium ou magnésium ;
- Y est choisi dans le groupe constitué par :
$C_1$-$C_3$ alkyle,
$CH_2OH$,
$CHOHCH_3$,
$COOC_1$-$C_3$ alkyle,
2-furyle ou $CH_2OCH_3$ ;

- Z est :
. un groupe hydrocarboné $C_2$-$C_{13}$, saturé ou insaturé, acyclique, cyclique ou mixte, ou
. un groupe hydrocarboné $C_2$-$C_{13}$ modifié, saturé ou insaturé, acyclique, cyclique ou mixte, dans lequel jusqu'à quatre C, CH ou $CH_2$ peuvent être remplacés par un à quatre hétéroatomes ou radicaux hétéroatomiques, identiques ou différents, choisis dans le groupe comprenant N, O, S ou NH, C étant remplacé par S, CH étant remplacé par N, et $CH_2$ étant remplacé par O, S ou NH ;
- R est un groupe hétérocyclique monocyclique ou bicyclique,
. le groupe monocyclique étant choisi dans le groupe constitué par les radicaux :

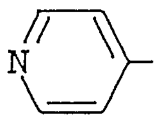

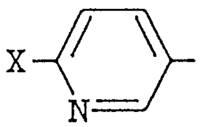

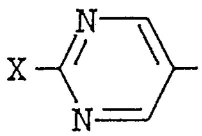

dans lesquels X est :
H,
Cl,
CN,
$COC_1$-$C_3$ alkyle,
$CONHC_1$-$C_3$ alkyle,
$COOC_1$-$C_3$ alkyle,
F,
$NO_2$,
$OCH_3$,
$SOC_1$-$C_3$ alkyle,
$SO_2C_1$-$C_3$ alkyle ou
$SO_2NHC_1$-$C_3$ alkyle,
. le groupe bicyclique étant choisi dans le groupe constitué par les radicaux :

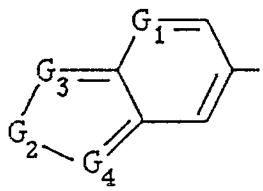

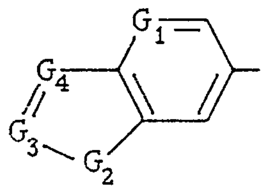

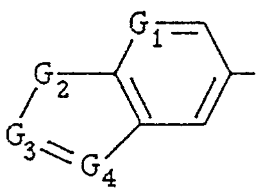

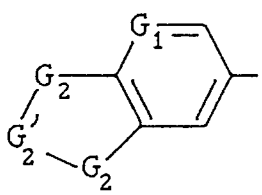

dans lesquels :
$G_1$ est CH ou N,
$G_2$ et $G_2'$ sont $CH_2$, CO, NH, $NCH_3$, O, S ou $SO_2$,
$G_3$ et $G_4$ sont CH, $CCH_3$, N ou NO ;
caractérisé en ce qu'il consiste à condenser un premier composé de formule :

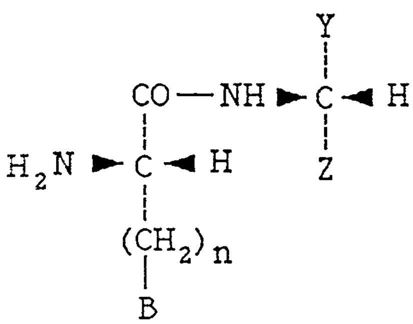

avec un second compose de formule :
R$-$N$=$C$=$A
dans laquelle A est O ou S,
ou un composé de formule :

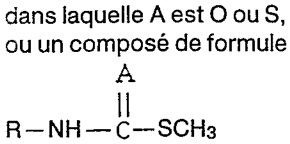

dans laquelle A est NCN,
dans lesquelles R, A, n, B, Y et Z répondent aux définitions données ci-dessus.

2. Procédé selon la revendication 1, caractérisé en ce que la réaction de condensation est effectuée

dans l'eau à température ambiante lorsque A est O ou S.

3. Procédé selon la revendication 1, caractérisé en ce que la réaction de condensation est effectuée à l'ébullition dans un mélange éthanol-eau lorsque A est NCN.

4. Procédé selon l'une des revendications 1 et 2, caractérisé en ce que dans les composés utilisés :

- R est un groupe 2-cyanopyrid-5-yle ou 2-cyanopyrimidin-5-yle ;
- A est 0 ;
- n est 1 ;
- Y est $COOCH_3$ ou $CH_3$ ;
- Z est un groupe butyle, pentyle, isobutyle, isopentyle, phényle, cyclohexyle, benzyle, cyclohexylméthyle, propylamide, 2-butylamide, dicyclopropylcarbinylamide ou 2,2,4,4-tétraméthylthiéthan-3-ylamide.

5. Procédé selon la revendication 1 pour la préparation d'un agent édulcorant de formule :

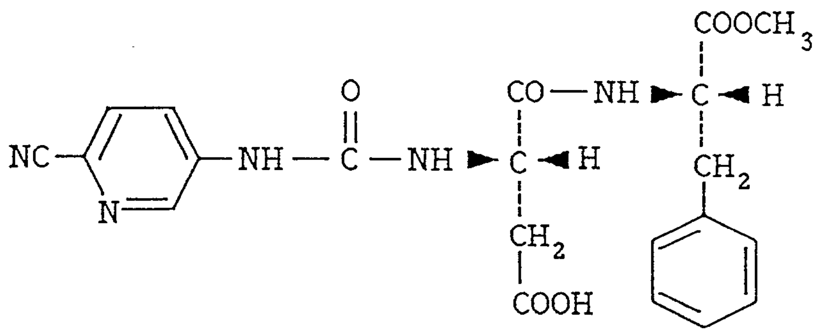

Office européen des brevets

# RAPPORT DE RECHERCHE EUROPEENNE

Numero de la demande

EP 88 42 0420

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | EP-A-0 195 731 (UNIVERSITE CLAUDE BERNARD)<br>* revendications *<br>--- | 1 - 7 | C 07 K 5/06<br>A 23 L 1/236 |
| A | EP-A-0 107 597 (UNIVERSITE CLAUDE BERNARD)<br>* revendications 2 - 7 *<br>----- | 1 - 3 | |
| | | | **DOMAINES TECHNIQUES RECHERCHES (Int. Cl.4)** |
| | | | C 07 K 5/00<br>A 23 L 1/00 |

Le présent rapport a été établi pour toutes les revendications

| Lieu de la recherche | Date d'achèvement de la recherche | Examinateur |
|---|---|---|
| BERLIN | 24-01-1989 | VAN AMSTERDAM L.J.P. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)